# EUROPEAN PATENT APPLICATION

(11) **EP 3 903 776 A1**
(43) Date of publication of application: **03.11.2021**
(21) Application number: 19904399.3
(22) Date of filing: 25.12.2019
(51) Int. Cl.: A61K 31/201, A61K 31/231, A61P 25/28

(54) **COMPOSITION FOR IMPROVING COGNITIVE FUNCTION**

(30) Priority: 25.12.2018 JP 2018241320
(71) Applicant: Demencare Laboratory Inc., Tokyo 101-0052 (JP)
(72) Inventor: NATORI, Shunji, Kitasoma-gun, Ibaraki 300-1622 (JP); KOMANO, Hiroto, Shiwa-gun, Iwate 028-3694 (JP); SUZUKI, Toshiharu, Chiba-shi, Chiba 260-0033 (JP); BAN, Saori, Sapporo-shi, Hokkaido 004-0863 (JP)
(74) Representative: Mewburn Ellis LLP
(86) International application number: PCT/JP2019/051030
(87) International publication number: WO 2020/138244

(57) **Abstract**

The purpose of the present invention is to provide a composition comprising conjugated linoleic acid for improving a cognitive function in an animal and to provide a composition for improving a cognitive function, comprising, as an active ingredient, c9,c11-conjugated linoleic acid (c9,t11-CLA)-containing conjugated linoleic acid (CLA) or a glyceride thereof.

## Description

### Technical Field

The present invention relates to a composition for improving a cognitive function.

### Background Art

Conjugated Linoleic Acid (CLA) is a linoleic acid isomer having a partial structure in which two carbon-carbon double bonds are conjugated (in a sequence such as -C=C-C=C-). Humans ingest, via, for instance, foods derived from ruminants, a tiny amount of CLA included in the foods.

Here, CLA is known to have beneficial effects on humans such as a body fat reducing effect, and foods and artificial supplements derived from organisms other than ruminants (for example, chicken meat and eggs), in addition to the foods derived from ruminants, have also been developed as CLA-containing products.

### Citation List

### Patent Literature

Patent Literature 1: International Publication No. WO 2018/186327

### Non Patent Literature

Non Patent Literature 1: Hunt W. T. et al. Protection of cortical neurons from excitotoxicity by conjugated linoleic acid. J. Neurochem. 115, 123-130 (2010) Non Patent Literature 2: Lee E, et al. Effect of conjugated linoleic acid, µ- calpain inhibitor, on pathogenesis of Alzheimer's disease. Biochim. Biophys. Acta 1831, 709-718 (2003)

### Summary of Invention

### Technical Problem

Meanwhile, one of the present inventors has previously found that, for example, lipids derived from larvae or pupae of *Sarcophaga peregrina* contain a large amount of c9,t11-CLA, an isomer of CLA. It has also been found that the purity of c9,t11-CLA in the lipids is several fold higher than the purity of c9,t11-CLA in lipids derived from ruminants, which purity has previously been considered to be the highest (see Patent Literature 1).

Here, it has been verified at a molecular level, a cellular level, or an organism level that administration of CLA to a mouse or rat elicits various physiological functions. Examples of the main physiological functions include a carcinogenesis suppressing effect, a body fat reducing effect, an anti-diabetic effect, an anti-arteriosclerotic effect, an immune enhancing effect, a bone metabolism improving effect, a blood pressure lowering effect, an anti-rheumatic effect, and an anti-inflammatory effect. Meanwhile, various theories have been proposed for the mechanism of action in which these physiological functions are expressed. However, the effects on Alzheimer's disease and/or the effects on other cognitive functions have just been examined at a molecular level or a cellular level. No research article has directly revealed the action upon *in vivo* administration. Thus, the action has yet to be verified at an organism level.

For example, Non-Patent Literature 1 is a research article reporting the results of examining the action of CLA on neurons in general at a molecular level or a cellular level. Non-Patent Literature 1 teaches that conventional CLA contains, as major isomers, c9,t11-CLA and t10,c12-CLA at a ratio of 1:1; and only c9,t11-CLA can exert the effects of preventing glutamic acid-induced hyper-excited state and cell death of neurons and can thus exert a neuroprotective effect. By contrast, t10,c12-CLA fails to exert such effects so that no neuroprotective effect can be elicited.

Some research articles have considered a specific neurological disease such as Alzheimer's disease and have reported the results of investigation on the effects of CLA on neurons. However, the investigated CLA has a low purity of c9,t11-CLA and contains, for instance, palmitic acid, stearic acid, oleic acid, and linoleic acid as well as CLA isomers in a large quantity (see Non-Patent Literature 2).

Unfortunately, it has not been revealed at all whether there exists CLA that has been considered so as to be able to treat or prevent a specific neurological disease such as Alzheimer's disease and is to improve cognitive functions in animals or, if any, what kind of compound the CLA is. In addition, how to deliver CLA to brain neurons in each animal is unclear. Thus, the behavior of CLA at an organism level cannot be speculated from the results of investigation at a molecular level or a cellular level. For example, to transfer CLA into the brain, permeability of CLA through the blood-brain barrier (BBB) has to be examined. Fatty acids are generally considered to be transferred via a transporter called Mfsd2a through the BBB to the brain. The permeability of trans fatty acids such as CLA has not been elucidated at all. Note that strictly speaking, examples of a chemical acting on brain neurons include not only those that have permeated through the blood-brain barrier, but also those in the blood staying in the brain. However, there have been no findings about the method capable of delivering an effective dose of trans fatty acids such as CLAto brain neurons for improving a cognitive function.

Here, the present invention has been made to solve the above problem in the prior art, and the purpose of the present invention is to provide a CLA-containing composition for improving a cognitive function in an animal.

### Solution to Problem

The present inventors have conducted intensive research to solve the above-mentioned problem in the prior art, and, as a result, have found that simple oral administration of a composition comprising, as an active ingredient, c9,t11-CLA-containing CLA or a triglyceride thereof can remarkably inhibit, in Alzheimer's disease model mice, formation of amyloid plaques, a pathological feature of the disease, and/or apoptosis of brain neurons.

Specifically, the present invention is as follows.
[1] A composition for improving a cognitive function, comprising, as an active ingredient, c9,t11-conjugated linoleic acid (c9,t11-CLA)-containing conjugated linoleic acid (CLA) or a glyceride thereof.
[2] The composition according to [1], wherein the composition comprises more than 80% by mass of total c9,t11-CLA, based on a total amount of total CLA.
[3] The composition according to [1] or [2], wherein the composition comprises 0.1 % or more by mass of the c9,t11-CLA based on a total amount of the composition.
[4] The composition according to any one of [1] to [3], wherein a mass ratio of total t10,c12-conjugated linoleic acid (t10,c12-CLA) to the total c9,t11-CLA is less than 0.25.
[5] The composition according to any one of [1] to [4], wherein a content of total t10,c12-CLA is less than 20 % by mass based on a total amount of the total CLA.
[6] The composition according to any one of [1] to [5], wherein the composition is for oral administration.
[7] A BACE1 inhibitor comprising the composition according to any one of [1] to [6].
[8] A food composition or feed composition for improving a cognitive function, comprising, as an active ingredient, c9,t11-CLA-containing conjugated linoleic acid or a glyceride thereof.
[9] The food composition or feed composition according to [8], wherein the improvement in the cognitive function is associated with suppressing formation of amyloid plaques or suppressing apoptosis of brain neurons.
[10] The food composition or feed composition according to [8] or [9], wherein the improvement in the cognitive function is associated with inhibiting BACE1.
[11] A pharmaceutical composition for use in the treatment or prevention of an impaired cognitive function, comprising, as an active ingredient, c9,t11-CLA-containing CLA or a glyceride thereof.
[12] The pharmaceutical composition according to [11], wherein the impaired cognitive function is a disease involving the formation of amyloid plaques or apoptosis of brain neurons.
[13] The pharmaceutical composition according to [11] or [12], wherein an improvement in the cognitive function is associated with BACE1.
[14] The pharmaceutical composition according to any one of [11] to [13], wherein the impaired cognitive function is Alzheimer dementia.
[15] An agent for treatment or prevention of an impaired cognitive function, comprising the pharmaceutical composition according to any one of [11] to [14].
[16] A method for treating or preventing an impaired cognitive function, comprising the step of administering the pharmaceutical composition according to any one of [11] to [14] to a subject in need thereof.

### Advantageous Effects of Invention

A composition for improving a cognitive function according to the invention can be used to make it possible to improve the cognitive function in an animal by simply orally administering to the animal the composition comprising, as an active ingredient, c9,t11-CLA-containing CLA or a triglyceride thereof.

Specifically, the composition for improving a cognitive function according to the invention can be used to improve the cognitive function in animals to treat and prevent an impaired cognitive function represented by Alzheimer's disease mainly because of having an activity of promoting formation of neurons in the whole brain including the hippocampus and cortex.

### Brief Description of Drawings

[Figure 1] Figure 1 is a graph showing the area of amyloid β protein 42 (Aβ42) deposition as identified and quantified from images of hippocampal Aβ42 deposition stained with an anti-Aβ42 antibody.
[Figure 2] Figure 2 is a graph showing the percentage of apoptotic neurons as quantified by the area of apoptotic neurons identified from staining images of hippocampal apoptotic neurons.
[Figure 3] Figure 3(A) is a graph showing quantification of wild-type mouse Aβ40 secreted into each culture medium. Figure 3(B) is the amino acid sequence of each of mouse or human Aβ1-40.
[Figure 4] Figure 4 is a graph showing quantification of BACE1 activity in each culture medium of wild-type mouse neurons.
[Figure 5] Figure 5(A) is a photographic image showing the results of analyzing the levels of APP, BACE1, PS1 NTF, and a membrane protein Flotillin in wild-type neurons by Western blot. Figure 5(B) is a graph showing quantification of the results in Figure 5(A).
[Figure 6] Figure 6 is a graph showing quantification of BACE1 activity in the presence or absence of LA or c9,t11-CLA while rBACE1 was used.
[Figure 7] Figure 7 is a graph showing quantification of wild-type mouse Aβ40 secreted into each culture medium.
[Figure 8] Figure 8 is photographs and a graph showing the results of observing, while AD model mice were used, IL-10-expressing cells in the cortex and hippocampus in the case of oral intake of c9,t11-CLA or in the case without intake.

### Description of Embodiments

Hereinafter, an embodiment of the present invention (hereinafter, referred to as the "present embodiment") will be described in detail. The present embodiment below is an example for explaining the present invention, and is not intended to limit the present invention to the following contents. The present invention can be exploited by appropriately modifying it within the scope of the gist.

A composition for improving a cognitive function according to the present embodiment (hereinafter, also referred to as a "composition of the present embodiment") comprises, as an active ingredient, c9,t11-conjugated linoleic acid (hereinafter, also referred to as "c9,t11-CLA")-containing conjugated linoleic acid (hereinafter, simply referred to as "CLA") or a glyceride thereof.

### [CLA]

CLA in the present embodiment (meaning those also including the below-described glyceride or sphingoid thereof) includes c9,t11-CLA. The mass ratio of t10,c12-CLA to c9,t11-CLA is less than 0.25. Here, "c", "t", and the following numerical values indicate whether the carbon-carbon double bond is either in a cis- or trans-configuration, and the position of the carbon forming the double bond counting from the carboxylic acid. For example, "c9,t11-CLA" indicates that the carbon-carbon double bonds are in a cis-configuration and a trans-configuration, and formed from carbons at positions 9 and 11, respectively, counting from the carbon of the carboxylic acid.

The CLA in the present embodiment is mainly used to be ingested by animals. That is, CLA in the present embodiment may be used to administer CLA that contains, as an active ingredient, c9,t11-CLA, a CLA isomer that exerts an effect of improving a cognitive function in an animal, and contains t10,c12-CLA in a relatively smaller amount.

The CLA in the present embodiment can be easily produced. This is because CLA having a mass ratio of t10,c12-conjugated linoleic acid (hereinafter, also referred to as "t10,c12-CLA") to c9,t11-CLA of less than 0.25 can be easily obtained, for example, by extraction from insects belonging to the order *Diptera* (see Patent Literature 1). The content disclosed in Patent Literature 1 is herein incorporated by reference in its entirety. It is possible to use, in the present embodiment, such a method for producing CLA, comprising extracting CLA from insects belonging to the order *Diptera.*

The CLA in the present embodiment means a linoleic acid isomer having a partial structure in which two carbon-carbon double bonds are conjugated (in a sequence such as -C=C-C=C-). In addition, those containing impurities such as other lipids are also included. CLA is a compound represented by C₁₈H₃₂O₂, and theoretically, 28 isomers exist. Specific examples of CLA isomers include c9,c11-CLA; c9,t11-CLA; t9,c11-CLA; t9,t11-CLA; c10,c12-CLA; c10,t12-CLA; t10,c12-CLA; t10,t12-CLA; and t11,t13-CLA.

Since CLA is produced from linoleic acid (n-6) in grass by the action of microorganisms present in the rumen of ruminants, it can be ingested through conventional foods derived from ruminants. However, the amount of CLA in such foods is extremely small, and is about 5 mg per g of fat even in those containing CLA in a large amount.

CLA products are commercially available in Japan and other countries as an anti-obesity supplement that focuses on the effect of reducing body fat of CLA. Commercially available CLA products are not extracted from natural materials, but artificially synthesized by alkaline isomerization of high linoleic acid safflower oil or the like. That is, a conventional CLA product as so artificially synthesized is, for instance, configured such that c9,t11-CLA is at 37.0 % by mass; t10,c12-CLA is at 38.4 % by mass; and the remainder such as other CLA isomers, palmitic acid, and oleic acid are included.

The physiological effects exhibited by the intake of CLA by animals have been verified mainly in animal experiments, and examples thereof include a carcinogenesis suppressing effect, a body fat reducing effect, an anti-diabetic effect, an anti-arteriosclerotic effect, an immune enhancing effect, a bone metabolism improving effect, a blood pressure lowering effect, and an anti-rheumatic effect. These beneficial physiological effects are mainly attributed to c9,t11-CLA, and some beneficial physiological effects (for example, body fat reducing effect) are attributed also to t10,c12-CLA. Thus, the investigation using conventional CLA with a large amount of isomers cannot reveal what kind of physiological effect can be exerted by c9,t11-CLA as an active ingredient.

Although t10,c12-CLA has some physiological effects in common with the above beneficial physiological effects (for example, a significant body fat reducing effect), it also has harmful physiological effects such as induction of hyperinsulinemia or fatty liver, carcinogenesis in breast cancer with erbB-2 gene overexpression, promotion of cancer metastasis, and organ enlargement, in addition to the beneficial effects on animals. In particular, when women take t10,c12-CLA for a long time as an anti-obesity supplement or the like, the possibility of an increased risk of developing breast cancer has been pointed out.

Next, examples of the effect of t10,c12-CLA on brain neurons include inhibition of growth of neural stem cells by t10,c12-CLA (Non-Patent Literature 3: Ham Wang et *al.* Isomer-specific effects of conjugated linoleic acid on proliferative activity of cultured neural progenitor cells. Mol. Cell. Biochem. 358. 13-20 (2011)). By contrast, c9,t11-CLA, which is used in a composition of the present embodiment and is an isomer of t10,c12-CLA, has been found to elicit an activity of promoting growth of neural stem cells, which activity is completely opposite to that of t10,c12-CLA. Note that if neural stem cells do not proliferate, no neurons are formed. Thus, c9,t11-CLA may be involved in the formation of neurons.

The glyceride or sphingoid in the present embodiment is a compound in which CLA is ester-bonded to the hydroxy group of glycerin (for example, triglyceride), or a compound in which CLA is amide-bonded or ester-bonded to the amino group or hydroxy group of sphingosine. Such compounds are obtained as an extract together with free CLA when, for example, CLA is derived from an insect described later. It is also possible to further extract only free CLA or only its glyceride or sphingoid from the obtained extract using a known method. The glyceride or sphingoid is not particularly limited as long as it has a skeleton forming the above-described ester bond or amide bond, and for example, may be a compound in which phosphoric acid or sugar is further bonded, or a compound forming a salt.

In the CLA in the present embodiment, the mass ratio of total t10,c12-CLA to total c9,t11-CLA (t10,c12/c9,t11) is less than 0.25, preferably 0.20 or less, more preferably 0.15 or less, further preferably 0.10 or less and furthermore preferably 0.05 or less.

The CLA in the present embodiment contains c9,t11-CLA, and preferably contains the total c9,t11-CLA in an amount of more than 80 % by mass, more preferably contains total c9,t11-CLA in an amount of 85 % by mass or more, further preferably contains the total c9,t11-CLA in an amount of 90 % by mass or more, furthermore preferably contains the total c9,t11-CLA in an amount of 95 % by mass or more and furthermore preferably contains the total c9,t11-CLA in an amount of 98 % by mass or more, based on the total amount of the total CLA (100 % by mass) .

The content of the total t10,c12-CLA in the CLA in the present embodiment is preferably less than 20 % by mass, more preferably less than 15 % by mass, further preferably less than 10 % by mass and furthermore preferably less than 5.0 % by mass, based on the total amount of the total CLA (100 % by mass).

In the present description, the "total c9,t11-CLA" means free c9,t11-CLA, and the triglycerides and sphingoids thereof, all of which are converted into free c9,t11-CLA. Regarding the "total t10,c12-CLA" and the "total CLA", similarly as the "total c9,t11-CLA", each means the free form, and the triglycerides and sphingoids thereof, all of which are converted into the free form.

A composition of the present embodiment comprises c9,t11-CLA in an amount of preferably 0.01 % by mass or larger and more preferably 0.1 % by mass or larger, based on the total amount of the composition.

### [Uses]

A composition comprising, as an active ingredient, the CLA and/or the glyceride and/or sphingoid thereof in the present embodiment is mainly used to improve a cognitive function in an animal. To realize the above, the composition can be provided as a food (in particular, a functional food) or feed used for improving a cognitive function or a medicament used for treating or preventing an impaired cognitive function. In each case, the composition of the present embodiment is a food composition, a feed composition, or a pharmaceutical composition. Examples of the "animal" include, but are not particularly limited to, mammals (e.g., a human, a mouse, a rat, a guinea pig, a rabbit, a dog, a horse, a monkey, a pig) and particularly humans. As used herein, the term "medicament" is a concept including a therapeutic agent and a prophylactic agent.

The food, feed, or pharmaceutical composition of the present embodiment contains the CLA in the above embodiment, and can further contain carriers and additives that are acceptable for foods, feeds, or medicaments.

Examples of the carriers and the additives include pharmaceutically acceptable organic solvents such as water, saline, phosphate buffer, dextrose, glycerol and ethanol, collagen, polyvinyl alcohol, polyvinyl pyrrolidone, carboxyvinyl polymer, sodium carboxymethyl cellulose, sodium polyacrylate, sodium alginate, watersoluble dextran, sodium carboxymethyl starch, pectin, methyl cellulose, ethyl cellulose, xanthan gum, gum arabic, casein, agar, polyethylene glycol, diglycerin, glycerin, propylene glycol, vaseline, paraffin, stearyl alcohol, stearic acid, human serum albumin, mannitol, sorbitol, lactose and surfactants, but are not limited thereto.

The food composition or feed composition of the present embodiment can be provided in the form of powder, capsule (hard capsule, soft capsule), microcapsule, syrup, pill, or tablet.

The pharmaceutical composition of the present embodiment may be in various forms depending on its administration route. Examples include powders, capsules (hard capsules, soft capsules), microcapsules, syrups, pills, tablets, liquids (e.g., injections), dispersions, suspensions, or suppositories.

The food composition or feed composition of the present embodiment may be used to improve a cognitive function. More specifically, the food composition or feed composition may be used to improve a cognitive function involving the suppression of the formation of amyloid plaques or the suppression of apoptosis in brain neurons, or a cognitive function involving the inhibition of BACE1. Here, β-site APP cleaving enzyme 1 is abbreviated as "BACE1" and is an aspartic protease for cleaving the β-cleavage site of APP in intracellular vesicles primarily such as endosomes or the Golgi apparatus. In addition, Amyloid Precursor Protein is abbreviated as APP and is an amyloid precursor protein that is a precursor of amyloid β contributing to the formation of amyloid plaques.

The pharmaceutical composition of the present embodiment may be used to treat or prevent an impaired cognitive function. More specifically, the pharmaceutical composition may be used to treat or prevent an impaired cognitive function involving formation of amyloid plaques or apoptosis of brain neurons or an impaired cognitive function involving BACE1. Among them, it is preferable to be used for treating or preventing Alzheimer dementia. As used herein, the "impaired cognitive function" means a brain neuron-related disorder.

The composition comprising, as an active ingredient, the CLA or a glyceride or sphingoid thereof according to the present embodiment may be a BACE1 inhibitor. It is particularly preferable that the composition is a BACE1 inhibitor in brain neurons.

Here, one can refer to the above Non-Patent Literature 2 about the relationship between the conventional CLA and neurons in animals. The composition of the present embodiment contains c9,t11-CLA in a higher purity enough to serve as an active ingredient than that of the conventional CLA. This makes it possible to improve a cognitive function and treat or prevent an impaired cognitive function.

### [Treatment or prevention method]

A method for treating or preventing an impaired cognitive function according to the present embodiment comprises the step of administering the pharmaceutical composition of the present embodiment to a subject in need thereof. As used herein, the "subject" is primarily the above-mentioned animal suffering from an impaired cognitive function and, in particular, a human patient with the impaired cognitive function.

As used herein, "treatment or prevention" means to produce at least one of cure or remission of the disease, as well as prevention or delay of the onset, prevention or delay of the progression of the disease, alleviation of at least one symptom relating to the disease.

In the method for administering to an animal a composition of the present embodiment, including the above-mentioned treatment or prevention method, any procedure may be used for administration as long as the administration can cause an effect on brain neurons in the animal. Examples include oral, nasal, transmucosal, vaginal, ocular, or intrarectal administration. A single oral dose of the composition of the present embodiment may be administered to exert an effect on brain neurons in an animal. Thus, the composition can be easily administered to a subject. This point is excellent.

### Examples

Hereinafter, the present invention is further described in detail with Examples, but the present invention is not limited to these Examples. Hereinafter, "%" means % by mass, unless otherwise specified.

### Example 1

### [Preparation of feed]

A regular mouse feed mixed with 2.0% (w/w) sunflower oil and 0.4% (w/w) c9,c-t11-CLA was provided as an experimental feed and a regular mouse feed mixed with only 2.4% (w/w) sunflower oil was provided as a control feed.
- Sunflower oil (trade name: "NIKKOL sunflower oil"; as fatty acid components, palmitic acid: 6.7%, oleic acid: 17.9%, stearic acid: 4.0%, linoleic acid (free of CLA): 69.8%, and linolenic acid: 0.9%; Wako Pure Chemical Industries, Ltd.).
- c9,t11-CLA (purity: 98%, ABCAM Inc.).
- Regular mouse feed (free of CLA).

### [Rearing of AD model mice]

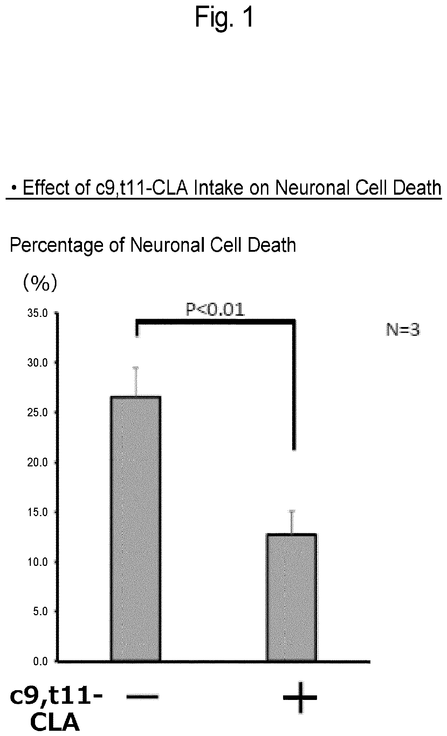

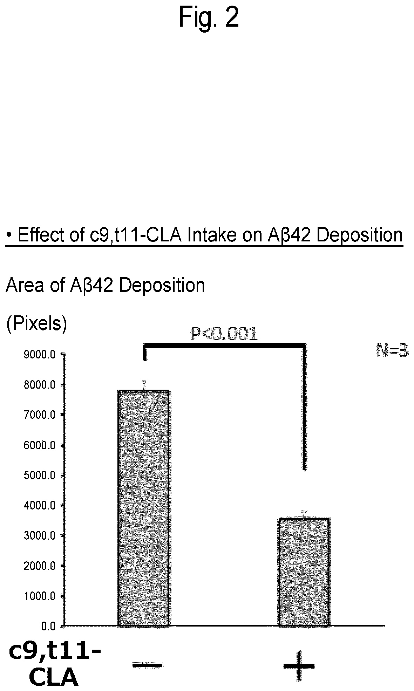

First, 7 male and 9 female mice (Jackson Laboratory, Inc.), in which human APP with Swedish and Indiana mutations was overexpressed, were reared with a regular mouse feed from the birth to 6 months after the birth, and were used as Alzheimer's disease (AD) model mice. The AD model mice were reared for 8 months while 3 male and 4 female mice were fed with the above experimental feed and 4 male and 5 female mice were fed with the above control feed. Then, the AD model mice were subject to autopsy to quantitatively determine amyloid plaques in the brain and the state of apoptosis of neurons. Figures 1 and 2 show the results.

Figure 1 is a graph showing the area of amyloid β protein 42 (Aβ42) deposition as identified and quantified from images of hippocampal Aβ42 deposition in each sample stained with an anti-Aβ42 antibody.
Figure 2 is a graph showing the percentage of apoptotic neurons as quantified by the area of apoptotic neurons identified from staining images of hippocampal apoptotic neurons in each sample.

As shown in Figures 1 and 2, the results of the AD model mice given the experimental feed demonstrated that the area of amyloid plaques in the hippocampal region in the brain and the percentage of apoptotic neurons were decreased markedly and were 50% or lower than those of the AD model mice given the control feed.

The above was the first to reveal unidentified characteristics such that oral administration of the composition comprising c9,t11-CLA-containing CLA as an active ingredient caused a decrease in formation of amyloid plaques in the AD model mice and further suppressed apoptosis of neurons in the hippocampus or cortex. Note that such effects are speculated to be exerted because of versatile layered molecular mechanisms.

### Example 2-1 (Inhibition of Aβ production and decrease in BACE1 activity by CLA)

Neurons in the cerebral cortex and hippocampus were isolated from the fetal brain of wild-type mouse (C57BL6/J) and were cultured in Neurobasal Medium containing 2% B-27 Supplement (Invitrogen), 4 mM Glutamax I, and 5% heat-inactivated horse serum (the other detailed conditions were substantially the same as in the protocol disclosed in Non-Patent Literature 4: Chiba et al., [2014] Mol. Biol. Cell 25, 3569-3580).

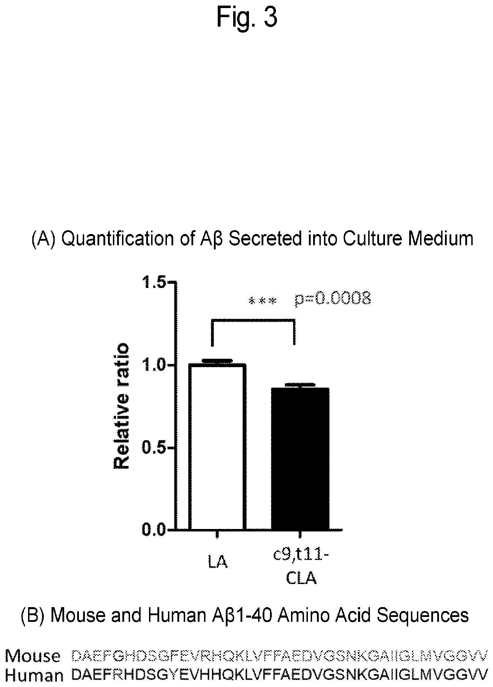

Primary culture nerves (5 × 10⁵ cells), which had been cultured by the above procedure for 9 to 12 days (DIV9-12), were cultured for 24 h in each of a culture medium containing 10 uM linoleic acid (LA "L1376", Sigma-Aldrich, Inc.) or a culture medium containing 10 uM c9,t11-CLA (purity: 98%, ABCAM, Inc.) (the experiment was triplicate: n = 3 to 6). Next, for each of a sample using LA or a sample using c9,t11-CLA, mouse amyloid β-protein 40 (Aβ40) secreted into the culture medium was quantified by sandwich ELISA (sELISA). The mouse Aβ40 was captured by an Aβ40 C-terminal fragment specific antibody 4D1 (described in Non-Patent Literature 5: Tomita et al., [1988] J. Biol. Chem. 273, 6277-6284). The complex was then reacted with an IgG Fab' fragment of HRP (Horseradish peroxidase)-labeled mouse/rat Aβ(1-16) sequence-specific (rabbit) antibody (IBL, Inc.: Immuno-Biological Laboratories Co, Ltd.). After that, TMB (3,3',5,5'-tetramethyl benzidine) was used as a chromogenic substrate for quantification. Figure 3(A) shows the results. In Figure 3(A), while the level of Aβ40 secreted when LA was added was set to 1.0, the level of Aβ40 secreted when c9,t11-CLA was added was quantified. The level of Aβ produced/secreted in the c9,t11-CLA-added neurons was significantly lower than that in the LA-added neurons.

The amount of Aβ42 generated from endogenous amyloid-β (Aβ) precursor protein APP is usually very small. Thus, it is difficult to accurately quantify Aβ42 secreted from wild-type mouse neurons. Then, the mouse Aβ sequence was changed to a human form (in Figure 3(B), the mouse and human amino acid sequences). After that, neurons from a knock-in mouse, into which a familial Alzheimer's disease mutation that increases the level of Aβ42 produced was introduced, (Apptm3.1Tcs/Apptm3.1Tcs) (hereinafter, the same as a mouse called APP-KI mouse described in Non-Patent Literature 6: Saito et al. [2014] Nature Neuroscience 17, 661-663) were cultured in a manner similar to the above. Finally, human Aβ42 secreted while the same experiments as in Figure 3(A) were repeated was quantified (Non-Patent Literature 7: Kimura, Hata, Suzuki [2016] J. Biol. Chem. 291, 24041-24053). Like in the wild-type mouse nerves, the level of human Aβ42 secreted from LA-treated nerves was significantly lower (p < 0.05) than that from c9,t11-CLA-treated nerves (not shown).

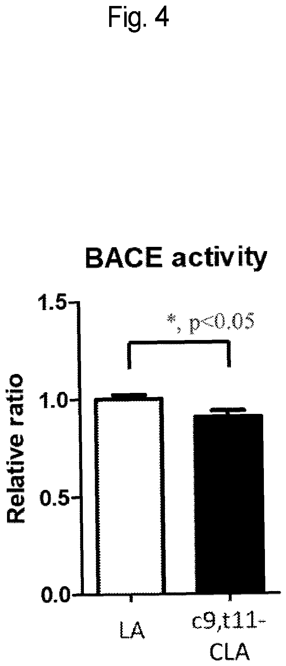

The BACE1 activity of the above wild-type mouse neurons used in the experiments shown in Figure 3 was measured using a β-Secretase (BACE1) Activity Assay Kit ("ab65357", Abcam, Inc.) in accordance with the instructions. Figure 4 shows the results. The BACE1 activity of the c9,t11-CLA-added neurons was significantly lower than that of the LA-added neurons. Similar results were obtained for the BACE1 activity of neurons from the above APP-KI mouse.

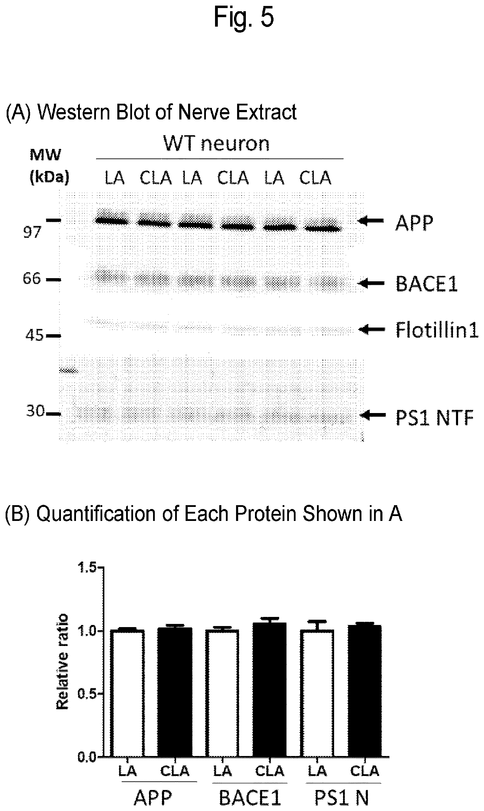

Western blot was used to analyze the level of APP (Aβ precursor, which is a substrate for BACE1), BACE1, PS1 NTF (a catalytic unit of γ-Secretase, which is an enzyme for cleaving CTFβ, an APP C-terminal fragment containing an Aβ sequence cleaved by BACE1, to generate Aβ), and a membrane protein Flotillin (control protein) in the LA-added or c9,t11-CLA-added wild-type neurons. The whole cells were lysed in RIPA buffer (components: 50 mM Tris-HCl [pH 8.0], 150 mM NaCl, 0.5% SDS, 0.5% sodium deoxycholate, and 1% Nonidet P-40). The extracted proteins (15 µg protein) were subjected to electrophoresis using SDS-polyacrylamide gel. Then, Western blot with each antibody was used to analyze APP (with the same as an antibody G369, described in Non-Patent Literature 8: Oishi et al. [1997] Mol. Med. 3, 111-123), BACE1 (with an antibody D10E5; Cell Signaling Technology, Inc.), PS1 NTF (with the same as an antibody Ab14, described in Non-Patent Literature 9: Thinakaran et al. [1996] Neuron, 17, 181-190), and Flotillin-1 (with an antibody 610821, BD Bioscience, Inc.). The protein reacted with each antibody was detected using a Clarity Western ECL substrate (Cat #170-5061; Bio-Rad) and quantified with LAS-4000 (Fujifilm). Figure 5 shows the results. There is no observed significant difference in the level of APP, BACE1, or PS1 NTF as well as the level of Flotillin between the case of using LA and the case of using c9,t11-CLA.

### Example 2-2 (c9,t11-CLA does not directly act on BACE1)

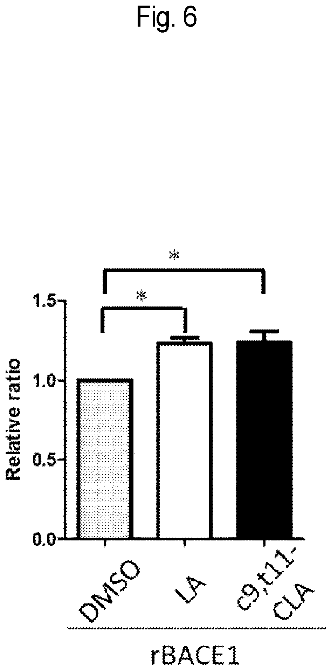

Whether or not c9,t11-CLA directly acted on BACE1 and changed its activity was checked by measuring the BACE1 activity in the presence or absence of LA or c9,t11-CLA while recombinant BACE1 (rBACE1, catalog number 931-AS; R&D Systems, Minneapolis, MN, USA) was used (Figure 6). First, 100 µM LA or c9,t11-CLA, which had been dissolved in DMSO, was added to rBACE1, and the mixture was incubated at 37°C for 15 min. Next, a chromogenic substrate was added and incubated at 37°C for 1 h. Then, the BACE1 activity was measured. As a result, LA or c9,t11-CLA caused an increase in the activity when compared to just a solvent DMSO. However, there was no observed significant difference in the activity between LA and c9,t11-CLA. This result has revealed that c9,t11-CLA does not directly act on BACE1. This seems to be because an increase in the activity by addition of LA or c9,t11-CLA causes stabilization of BACE1, a membrane protein, by the addition of lipids.

Figure 3 shows the results in which c9,t11-CLA acts on neurons to inhibit Aβ secretion. One of causes for a decrease in amyloid plaques in the c9,t11-CLA-administered mice of the AD mouse model may involve that c9,t11-CLA functions to decrease production of Aβ. Further, Figure 4 shows the results showing a decrease in the BACE1 activity of the c9,t11-CLA-added neurons. The decrease in Aβ may be caused because c9,t11-CLA causes a decrease in the BACE1 activity.

The results obtained from Figure 5 have demonstrated that c9,t11-CLA does not affect the level of a substrate APP, an APP-cleaving enzyme BACE1, or PS1 NTF in the neurons. In addition, Figure 6 shows that neither LA nor c9,t11-CLA directly acts on the BACE1 activity. This is a novel finding indicating that c9,t11-CLA acts on neurons and indirectly reduces an APP cleavage by BACE1 to inhibit production of Aβ. Compounds which directly inhibit the BACE1 activity have not been put into practice since the compounds also suppress cleavage of other substrates and produce side effects. Here, c9,t11-CLA does not directly act on BACE1 but reduces the BACE1 activity. This makes it possible to suppress production of Aβ. As a result, this substance can be understood as having a novel AD-suppressing effect of suppressing AD pathology.

### Example 2-3

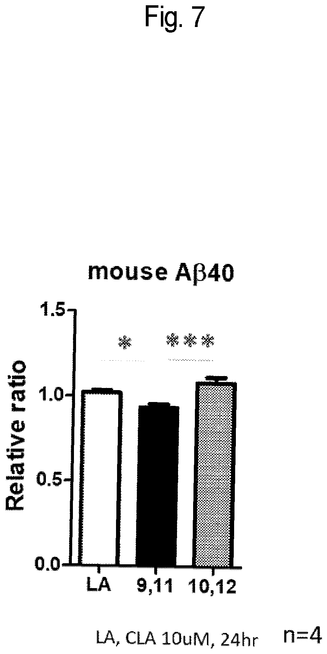

A culture medium containing 10 uM t10,c12-CLA was added, and each mixture was cultured for 48 h to prepare each sample. Except for that, substantially the same procedure as in Example 2-1 was repeated. Figure 7 show the results like in Figure 3(A).

### Example 3

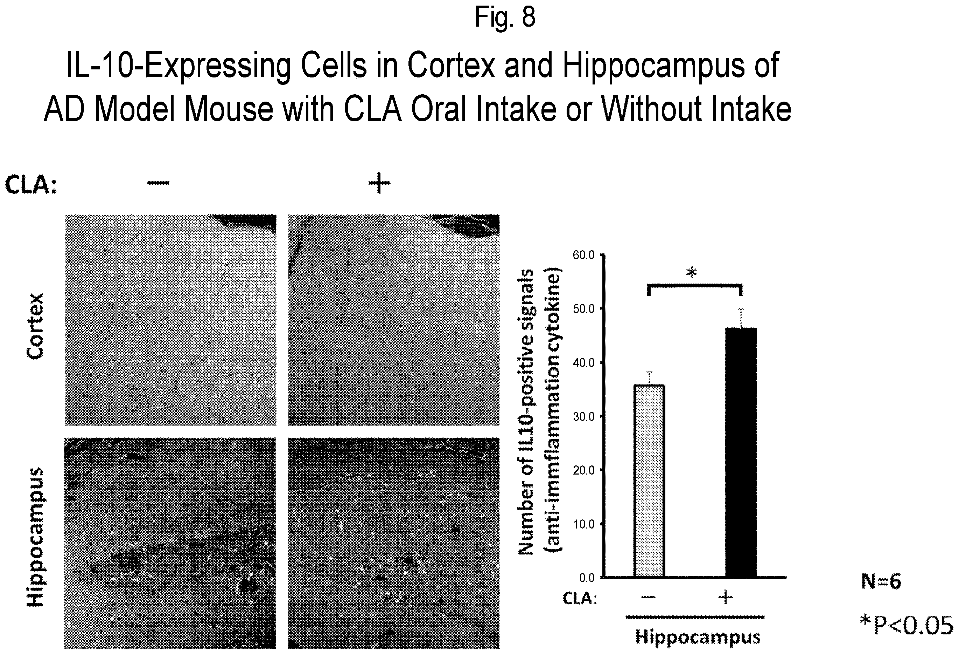

While substantially the same AD model mice as in Example 1 were used, IL-10-expressing cells in the cortex and hippocampus were observed in the case of oral intake of c9,t11-CLA or in the case without intake. Figure 8 show the results.

The results of Figure 8 show an increase in the number of IL-10-producing cells in the hippocampus, suggesting an increase in an anti-inflammatory cytokine IL-10.
- Phosphorylated tau protein staining: although no significant difference was detected, the staining tended to decrease in the hippocampus.
- Astrocyte staining: although no significant difference was detected, the staining tended to decrease in the hippocampus.
- Microglia (Iba1 staining): in the CLA intake group, there was a significant increase in both the hippocampus and the cortex.
- Anti-inflammatory cytokine IL-10: the number of IL-10-expressing cells significantly increased in the hippocampus in the CLA intake group.

As described above, the intake of c9,t11-CLA causes an increase in activated microglia and an increase in the number of IL-10-producing cells in the hippocampus. Thus, the anti-inflammatory cytokine IL-10 is considered to increase.

### Industrial Applicability

The composition according to the invention is useful in the fields of food (for example, food for humans and food for pets, in particular functional food), feed (for example, feed for domestic animals), medicaments (for example, medicaments for human and medicaments for pets), and the like.

## Claims

1. A composition for improving a cognitive function, comprising, as an active ingredient, c9,t11-conjugated linoleic acid (c9,t11-CLA)-containing conjugated linoleic acid (CLA) or a glyceride thereof.

2. The composition according to claim 1, wherein the composition comprises more than 80% by mass of total c9,t11-CLA, based on a total amount of total CLA.

3. The composition according to claim 1 or 2, wherein the composition comprises 0.1 % or more by mass of the c9,t11-CLA based on a total amount of the composition.

4. The composition according to any one of claims 1 to 3, wherein a mass ratio of total t10,c12-conjugated linoleic acid (t10,c12-CLA) to the total c9,t11-CLA is less than 0.25.

5. The composition according to any one of claims 1 to 4, wherein a content of total t10,c12-CLA is less than 20 % by mass based on a total amount of the total CLA.

6. The composition according to any one of claims 1 to 5, wherein the composition is for oral administration.

7. A BACE1 inhibitor comprising the composition according to any one of claims 1 to 6.

8. A food composition or feed composition for improving a cognitive function, comprising, as an active ingredient, c9,t11-CLA-containing conjugated linoleic acid or a glyceride thereof.

9. The food composition or feed composition according to claim 8, wherein the improvement in the cognitive function is associated with suppressing formation of amyloid plaques or suppressing apoptosis of brain neurons.

10. The food composition or feed composition according to claim 8 or 9, wherein the improvement in the cognitive function is associated with inhibiting BACE1.

11. A pharmaceutical composition for use in the treatment or prevention of an impaired cognitive function, comprising, as an active ingredient, c9,t11-CLA-containing CLA or a glyceride thereof.

12. The pharmaceutical composition according to claim 11, wherein the impaired cognitive function is a disease involving formation of amyloid plaques or apoptosis of brain neurons.

13. The food composition or feed composition according to claim 11 or 12, wherein an improvement in the cognitive function is associated with BACE1.

14. The pharmaceutical composition according to any one of claims 11 to 13, wherein the impaired cognitive function is Alzheimer dementia.

15. An agent for treatment or prevention of an impaired cognitive function, comprising the pharmaceutical composition according to any one of claims 11 to 14.

16. A method for treating or preventing an impaired cognitive function, comprising the step of administering the pharmaceutical composition according to any one of claims 11 to 14 to a subject in need thereof.
